# EUROPEAN PATENT SPECIFICATION

(11) **EP 4 104 188 B1**
(45) Date of publication and mention of the grant of the patent: **05.06.2024**
(21) Application number: 21710660.8
(22) Date of filing: 09.02.2021
(51) Int. Cl.: G07F 17/00, G16H 20/13, G16H 40/63

(54) **DIVERSION DETECTION SYSTEM**
UMLENKUNGSERKENNUNGSSYSTEM
SYSTÈME DE DÉTECTION DE DÉTOURNEMENT

(30) Priority: 11.02.2020 US 202062975056 P
(43) Date of publication of application: 21.12.2022
(73) Proprietor: Carefusion 303 Inc., San Diego, California 92130 (US)
(72) Inventor: BURGESS, Brendan, San Diego, California 92130 (US); YUSUFI, Mustafa, San Diego, California 92130 (US); FELKE, Magnus, San Diego, California 92130 (US); PREZIOTTI, Paul, San Diego, California 92130 (US); LATORRACA, Gary, San Diego, California 92130 (US)
(74) Representative: Schulz, Oliver Frank Michael
(86) International application number: PCT/US2021/017242
(87) International publication number: WO 2021/163051

(56) References cited:
- US-A1- 2013 070 090
- US-A1- 2019 244 699

## Description

### CROSS-REFERENCE TO RELATED APPLICATION

The present application claims priority to U.S. Provisional Application No. 62/975,056, filed on February 11, 2020, and entitled "Diversion Detection System".

### TECHNICAL FIELD

The subject matter described herein relates generally to the management of pharmaceuticals and more specifically to a system for a diversion detection system.

### BACKGROUND

Diversion may refer to the transfer of a controlled and/or high-value substance to a third party who is not legally authorized to receive, possess, and/or consume the substance for personal use or personal gain. High-value and/or controlled prescription medications, notably opiates, opioids, and narcotics, may be especially prone to diversion. For instance, a prescription pain medication may be diverted when a clinician keeps the prescription pain medication for unauthorized personal use instead of administering the prescription pain medication to a patient or wasting the prescription pain medication. Generally, generating evidence of the diversion of medication and identifying the diversion may be difficult, as cameras may merely record hours of footage without context, and without clearly showing the actual diversion of medication. Such systems may be unreliable, difficult to maintain, expensive, and require a significant amount of device resources (e.g., power, processing time, memory, network bandwidth, and the like).

From US 2019/0244699 A1 a system for dispensing medication is known, wherein a diversion may be detected based on data received from a dispensing cabinet and by applying a machine learning model trained to detect diversion.

Furthermore, US 2013/0070090 A1 discloses a system for monitoring the stocking and dispensing of medications based on imaging data and provides a synchronization of camera image data to facilitate investigation.

### SUMMARY

The scope of the invention is defined by independent claim 1. Preferred embodiments are depicted in the dependent claims, the Figures and the description. Accordingly, systems are provided for preventing the diversion of medication using one or more surveillance devices to capture transactions involving the medication and a diversion controller to generate easily sortable evidence of diversion that corresponds to the captured transactions.

A method implemented by such system is provided. The method includes detecting a triggering event at at least one dispensing cabinet including medication. The method also includes activating, in response to the detection of the triggering event, one or more surveillance devices to capture a recording comprising a possible diversion event at the at least one dispensing cabinet. At least one of the one or more surveillance devices may have an area of detection that includes the at least one dispensing cabinet. The method also includes generating one or more transaction records associated with the recording. The method also includes linking the one or more transaction records to at least a portion of the recording that comprises the captured possible diversion event. The method further includes filtering, based on the one or more transaction records, a plurality of recordings comprising the recording, to determine whether the possible diversion event occurred. The method also includes in response to a determination that the possible diversion event occurred, generating an alert indicating that the possible diversion event has occurred.

The linking includes linking the one or more transaction records with one or more of a timestamp indicating a start time and an end time of the triggering event during at least the portion of the recording, and preferably a surveillance device identifier of a surveillance device of the one or more surveillance devices that captured at least the portion of the recording.

In some aspects, the method also includes storing, at the at least one dispensing cabinet, the linked one or more transaction records and at least the portion of the recording.

In some aspects, the plurality of recordings are filtered at a database remote from the at least one dispensing cabinet.

In some aspects, the method also includes accessing, at a database remote from the at least one dispensing cabinet, the one or more transaction records and the recording captured by the one or more surveillance devices.

In some aspects, the method also includes determining, based on the filtered transaction records and the recording, that the possible diversion event has occurred.

In some aspects, the triggering event includes one or more of: an individual entering a patient room, an individual being located within a predetermined proximity of the at least one dispensing cabinet for a threshold amount of time, and an individual interacting with the at least one dispensing cabinet.

In some aspects, the recording comprises at least one video, image, and audio recording.

In some aspects, the one or more surveillance devices comprises a first surveillance device positioned on the at least one dispensing cabinet and a second surveillance device positioned on an auxiliary device coupled with the at least one dispensing cabinet. In some aspects, the captured possible diversion event is transmitted from an auxiliary surveillance device hub at the auxiliary device to a main surveillance device hub at the at least one dispensing cabinet.

The one or more transaction records comprises one or more of a clinician identifier, a patient identifier, a transaction type, a transaction identifier, a witness identifier, and a medication type.

In some aspects, the method also includes detecting, based on the recording, a quantity of the medication retrieved from the at least one dispensing cabinet. The determination that the possible diversion event occurred may include determining that the detected quantity of the medication is different from an expected quantity of the medication.

Implementations of the current subject matter can include methods consistent with the descriptions provided herein as well as articles that comprise a tangibly embodied machine-readable medium operable to cause one or more machines (e.g., computers, etc.) to result in operations implementing one or more of the described features. Similarly, computer systems are also described that may include one or more processors and one or more memories coupled to the one or more processors. A memory, which can include a non-transitory computer-readable or machine-readable storage medium, may include, encode, store, or the like one or more programs that cause one or more processors to perform one or more of the operations described herein. Computer implemented methods consistent with one or more implementations of the current subject matter can be implemented by one or more data processors residing in a single computing system or multiple computing systems. Such multiple computing systems can be connected and can exchange data and/or commands or other instructions or the like via one or more connections, including, for example, to a connection over a network (e.g. the Internet, a wireless wide area network, a local area network, a wide area network, a wired network, or the like), via a direct connection between one or more of the multiple computing systems, etc.

FIG. 6 depicts an example of a database showing one or more transaction records associated with a recording of a surveillance system, in accordance with some example embodiments;

FIG. 7 depicts a flowchart illustrating a process for generating evidence of diversion, in accordance with some example embodiments; and

FIG. 8 depicts a block diagram illustrating a computing system, in accordance with some example embodiments.

When practical, similar reference numbers denote similar structures, features, or elements.

### DETAILED DESCRIPTION

A possible diversion event including the diversion of a medication may occur at any point in time during the compounding, dispensing, administration, and/or wasting of the medication. For example, diversion may take many forms, including the removal of more doses than are permitted to be dispensed, removal of a dose and replacement with an illegitimate dose, replacement of a liquid controlled substance with saline or other liquids, and/or the like. Prescription pain medications such as, for example, opioids, morphine, hydromorphone, fentanyl, and/or the like, may be especially prone to diversion due to a lack sufficient custodial oversight during the dispensing, administration, and wasting of the prescription pain medication. For example, dispensing cabinets, such as automated dispensing cabinets, at medical facilities may be accessible to multiple clinicians. Moreover, different clinicians may be responsible for the dispensing, administration, and wasting of the medication. Thus, even when diversion is detected, it may be difficult to determine when the diversion actually occurred, obtain evidence that diversion actually occurred, and identify the individuals responsible for the diversion.

Cameras connected to medical facility security systems may generally be used to record images, video, and/or audio of a room at the medical facility, allowing for a security team to sift through the recorded images, video, and/or audio. However, such systems do not provide useful information to the security team about the individuals who accessed the dispensing cabinet in the room, what was accessed at the dispensing cabinet, what should have been accessed and/or wasted at the dispensing cabinet, and/or the like. Such systems may also require the security team to review thousands of hours of security footage to determine who entered the particular room, without providing useful information to the security team. Furthermore, the cameras may be positioned such that they are unable to provide a clear image of exactly who accessed the dispensing cabinet, what was taken from the dispensing cabinet, whether a correct amount of medication was taken from and/or wasted at the dispensing cabinet and/or the like.

The diversion detection system described herein may desirably generate useful evidence of the diversion of medication that may help to more easily identify the individual responsible for the diversion. For example, the diversion detection system described herein may use a surveillance system having one or more surveillance devices to capture one or more diversion events. The diversion detection system described herein may also include a diversion controller to generate one or more transaction records associated with the diversion event. The captured diversion and associated transaction records may be transmitted to and be stored at a dispensing cabinet, and/or be transmitted to and be stored at a database to be later filtered to provide evidence of the diversion event. The transaction records may also be linked to the diversion event. Linking the one or more transaction records with at least a portion of the recording that includes the captured diversion event allows for each of the portions of the recording to be filtered more easily by the associated transaction records. This may reduce the required device resources (e.g., power, processing time, memory, network bandwidth, and the like) to determine whether a diversion event occurred. For example, such configurations may help to more quickly sort the transaction records and/or a relevant portion of the recording, to quickly and/or efficiently determine whether a diversion occurred and if the diversion event occurred, the individual, medication, and/or patient associated with the diversion event. Accordingly, linking the transaction records with at least the portion of the recording that includes the possible diversion event may significantly reduce the amount of time it takes to identify that a diversion has occurred and the clinician or patient involved with the diversion, prevent the diversion event from occurring or continuing, and/or reduce the likelihood that the diversion will occur.

In some example embodiments, a diversion detection system may be configured to monitor and/or track access to the medication stored in a dispensing cabinet by at least capturing data associated with individuals who access the dispensing cabinet, for example, to retrieve medication and/or return unused medication, and/or data associated with an actual or possible diversion event. For example, the diversion detection system may capture images, videos, and/or audio recordings from one or more surveillance devices, of the individuals accessing the dispensing cabinet, of the medication being retrieved and/or wasted at the dispensing cabinet, and/or the like. The diversion detection system may also perform a biometric scan to capture, for example, a fingerprint, iris pattern, retina pattern, handwritten signature, voice, and/or the like, of the individuals accessing the dispensing cabinet. Alternatively and/or additionally, the diversion detection system may be configured to receive an identification number and/or a passcode that uniquely identifies the individuals accessing the dispensing cabinet. Alternatively and/or additionally, the diversion detection system may be configured to generate one or more transaction records associated with the captured images, videos, and/or audio recordings.

By capturing data from the one or more surveillance devices, linking the data to the captured recordings from the one or more surveillance devices, and storing the data locally at the dispensing cabinet and/or transmitting the data to a database to be later filtered and/or accessed, the diversion detection system may generate evidence of the diversion event to help to more easily identify that a diversion event has occurred and/or identify the individuals performing the diversion. The diversion detection system described herein may also help to identify the individual responsible for the diversion when a diversion event has been detected in real-time or after the diversion event occurred. The diversion detection system described herein may additionally and/or alternatively help to detect whether a diversion event has occurred by determining whether a discrepancy exists in the medication retrieved from the dispensing cabinet (e.g., an incorrect amount of medication is returned and/or wasted at the dispensing cabinet, and/or the like).

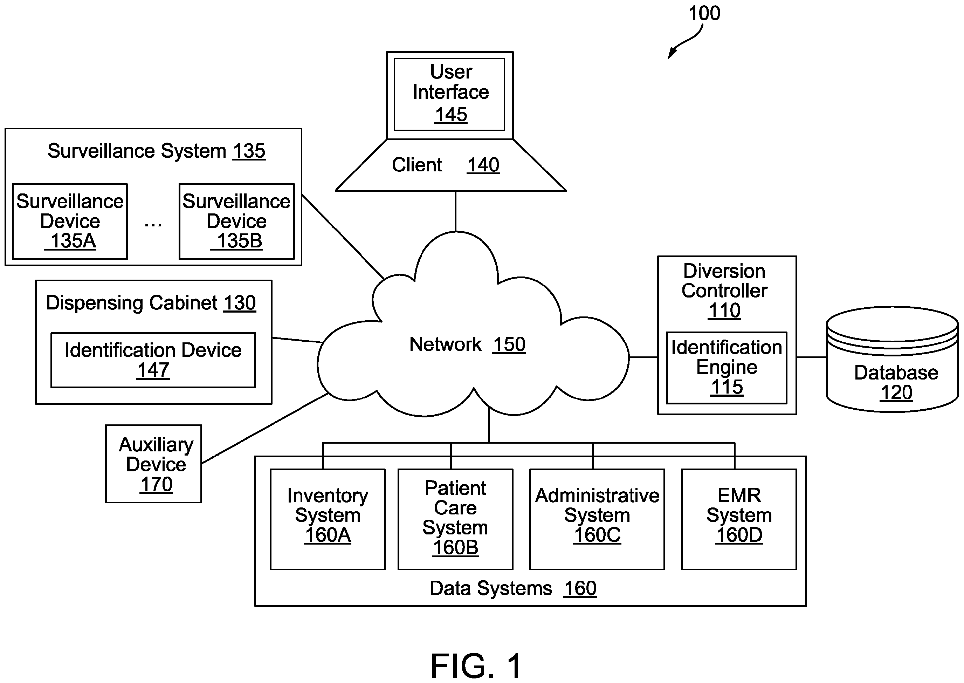

FIG. 1 depicts a system diagram illustrating a diversion detection system 100, in accordance with some example embodiments. The diversion detection system 100 may include a diversion controller 110, at least one dispensing cabinet 130, an auxiliary device 170, a surveillance system 135, one or more data systems 160, and a client 140. As shown in FIG. 1, the diversion controller 110, the dispensing cabinet 130, the auxiliary device 170, the surveillance system 135, the one or more data systems 160, and the client 140 may be communicatively coupled via a network 150. The diversion controller 110 may be coupled to or otherwise form a part of the dispensing cabinet 130, the auxiliary device 170, and/or the surveillance system 135. In some embodiments, the client 140 may be coupled to or otherwise form a part of the dispensing cabinet 130, the auxiliary device 170, and/or the surveillance system 135. The network 150 may be any wired and/or wireless network including, for example, a public land mobile network (PLMN), a wide area network (WAN), a local area network (LAN), a virtual local area network (VLAN), the Internet, and/or the like.

As shown in FIG. 1, the dispensing cabinet 130 may include an identification device 147. The identification device 147 may be a biometric reader configured to capture, for example, a fingerprint, iris pattern, retina pattern, handwritten signature, voice, and/or the like, of the individuals accessing the dispensing cabinet 130. Alternatively and/or additionally, the identification device 147 may be configured to receive the identification numbers and/or passcodes of the individuals accessing the dispensing cabinet 130 via, for example, a keyboard and/or a keycard scanner. It should be appreciated that the keycard scanner may obtain this data (e.g., the identification numbers and/or passcodes) in any manner including, for example, near-field communication (NFC), radio frequency identification (RFID), barcodes, quick response (QR) codes, and/or the like.

Referring again to FIG. 1, the surveillance system 135 may include one or more surveillance devices, such as a first surveillance device 135A and a second surveillance device 135B, which may be any recording device including, for example, a video camera, a still image camera, an audio recorder, and/or the like. The one or more surveillance devices may have an area of detection that includes the dispensing cabinet 130. In some embodiments, the surveillance system 135 also includes a motion sensor coupled with one or more of the surveillance devices. Additionally and/or alternatively, the surveillance devices may be capable of recording images and/or video in the dark or other low-light environments. The first and second surveillance devices 135A, 135B may be coupled to, be positioned on, or otherwise form a part of the dispensing cabinet 130, the auxiliary device 170, which may include one or more auxiliary devices coupled with (e.g., via a wired or wireless connection) the dispensing cabinet 130, or a room of the patient. The surveillance system 135 may be configured to capture images, videos, and/or audio recordings of individuals accessing the dispensing cabinet 130, for example, to retrieve and/or return medication.

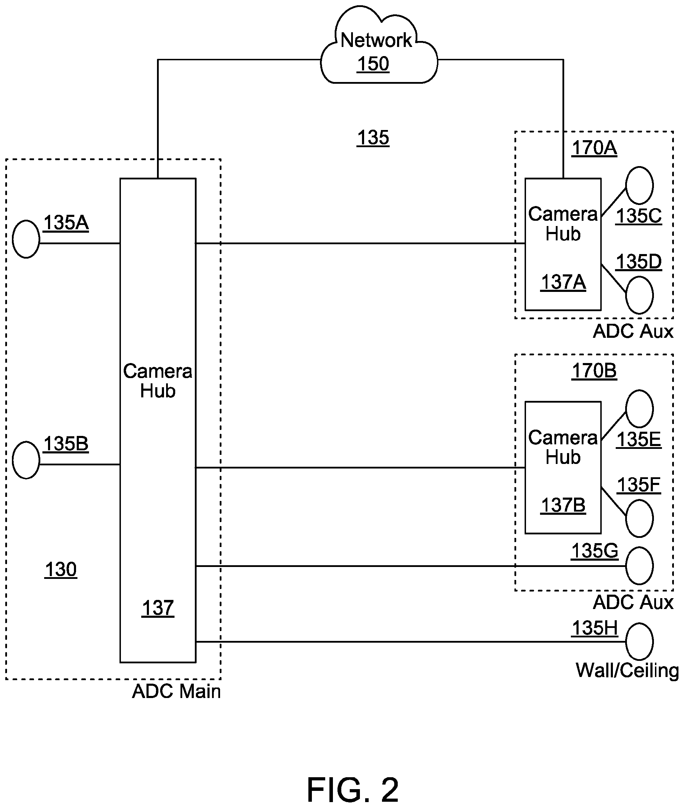

To further illustrate, FIG. 2 illustrates an example of the surveillance system 135 consistent with implementations of the current subject matter. As shown in FIG. 2, the surveillance system 135 may include a first surveillance device 135A, a second surveillance device 135B, a third surveillance device 135C, a fourth surveillance device 135D, a fifth surveillance device 135E, a sixth surveillance device 135F, a seventh surveillance device 135G, and an eighth surveillance device 135H. It should be appreciated that the surveillance system 135 may include one, two, three, four, five, six, seven, eight, nine, or ten or more surveillance devices. As shown in FIG. 2, the first and second surveillance devices 135A, 135B are positioned on the dispensing cabinet 130 and are coupled to a main surveillance device hub 137. The first and second surveillance devices 135A, 135B may capture a clinician's face and/or hands at the dispensing cabinet 130. In this example, the first and second surveillance devices 135A, 135B may capture recordings (e.g., video, audio, and/or still images) and the diversion controller 110 (e.g., the detection engine 115) may generate one or more transaction records associated with the recordings. The detection engine 115 may transmit the recordings and/or the associated transaction records from the first and second surveillance devices 135A, 135B to the main surveillance device hub 137 of the dispensing cabinet 130.

In the example shown in FIG. 2, the auxiliary device 170 includes a first auxiliary device 170A and a second auxiliary device 170B, which may be coupled to the dispensing cabinet 130 via a wired or wireless connection (e.g., via the network 150). The first auxiliary device 170A may include a first auxiliary surveillance device hub 137A, which may communicate with the main surveillance device hub 137 via the network 150. The first auxiliary device 170A may include the third and fourth surveillance devices 135C, 135D, which may capture recordings and the detection engine 115 may generate one or more transaction records associated with the recordings. The detection engine 115 may transmit the recordings and/or the associated transaction records from the third and fourth surveillance devices 135C, 135D to the first auxiliary surveillance device hub 137A. From the first auxiliary surveillance device hub 137A, the detection engine 115 may transmit the recordings and/or the associated transaction records to the main surveillance device hub 137.

In some embodiments, the second auxiliary device 170B may include a second auxiliary surveillance device hub 137B, which may, like the first auxiliary surveillance device hub 137A, communicate with the main surveillance device hub 137 via the network 150. The second auxiliary device 170B may include the fifth, sixth, and seventh surveillance devices 135E, 135F, 135G, which may capture recordings. The detection engine 115 may generate one or more transaction records associated with the recordings from the fifth and sixth surveillance devices 135E, 135F and/or the associated transaction records from the fifth and sixth surveillance devices 135E, 135F to the second auxiliary surveillance device hub 137B. From the second auxiliary surveillance device hub 137B, detection engine 115 may transmit the recordings and/or the associated transaction records to the main surveillance device hub 137. In some embodiments, however, the detection engine 115 may transmit the recordings and/or the associated transaction records from the seventh surveillance device 135G (or one or more of the other surveillance devices) directly to the main surveillance device hub 137. In some embodiments, the eighth surveillance device 137H may be positioned at another location in the room of the patient, separate from one of the auxiliary devices 170. The detection engine 115 may transmit the recordings and/or the associated transaction records from the eighth surveillance device 135H directly to the main surveillance device hub 137, via the network 150.

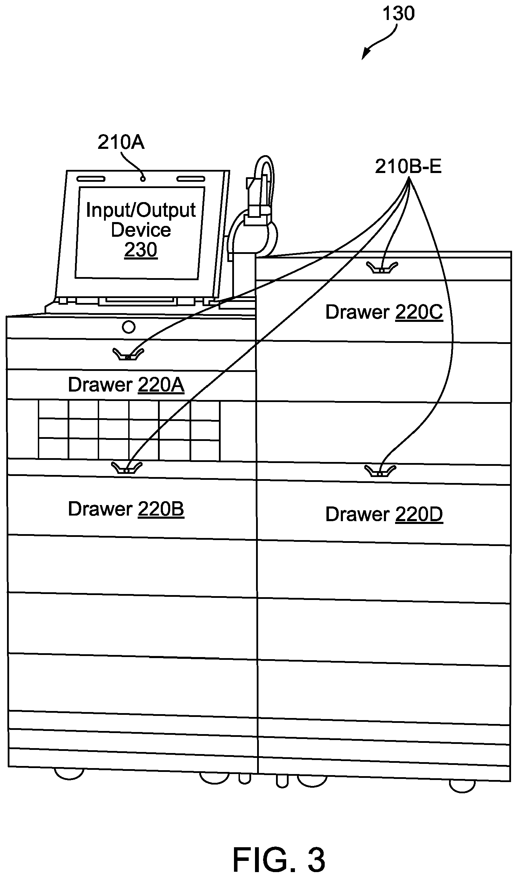

FIG. 3 depicts the dispensing cabinet 130, in accordance with some example embodiments. Referring to FIG. 3, the dispensing cabinet 130 may include a plurality of drawers including, for example, a first drawer 220A, a second drawer 220B, a third drawer 220C, a fourth drawer 220D, and/or the like. The surveillance system 135 of the dispensing cabinet 130 may include a plurality of cameras including, for example, a first camera 210A, a second camera 210B, a third camera 210C, a fourth camera 210D, a fifth camera 210E, and/or the like, which may be the same or similar to the surveillance devices described above with respect to FIG. 2. Meanwhile, the dispensing cabinet 130 may further include an input/output device 230, which may include a keyboard, a monitor, a touchscreen, and/or the like. It should be appreciated that the input/output device 230 may be part of the identification device 135B, and/or the client 140. For example, an individual accessing the dispensing cabinet 130 may enter, via the input/output device 230, an identification number and/or a passcode that uniquely identifies the individual. Moreover, the dispensing cabinet 130 may include a different quantity of cameras and/or drawers than shown.

As shown in FIG. 3, each camera in the surveillance system 135 may have a different field of view, and may be activated upon detection of one or more triggering events. For example, the first camera 210A may be a front facing camera configured to capture images, audio, and/or videos that include the face of the individuals who access the dispensing cabinet 130. The first camera 210A may be activated when an individual interacts with the input/output device 230. Meanwhile, the second camera 210B, the third camera 210C, the fourth camera 210D, and the fifth camera 210E may be downward facing cameras configured to capture images and/or videos of the interiors of the corresponding drawers. For example, the second camera 210B may be activated in response to the first drawer 220A being opened. The second camera 210B may further capture images and/or videos of medication being removed from and/or returned to the first drawer 220A. Alternatively and/or additionally, the third camera 210C may be activated in response to the second drawer 220B being opened. The third camera 210C may capture images and/or videos of medication being removed from and/or returned to the second drawer 220B.

In some example embodiments, the first camera 210A, the second camera 210B, the third camera 210C, the fourth camera 210D, and/or the fifth camera 210E may be activated upon detection of a triggering event (discussed in more detail below), such as whenever an individual accesses the dispensing cabinet 130. For example, when an individual accesses the dispensing cabinet 130 and returns unused medication to the first drawer 220A, the second camera 220B may be activated in order to capture images and/or videos of the individual returning the unused medication to the first drawer 220A. These images and/or videos may provide evidence of diversion including, for example, efforts to tamper with the unused medication. Alternatively and/or additionally, the first camera 220A may also be activated in order to capture images and/or videos that includes the face of the individual. These images and/or videos, and associated transaction records generated by the detection engine 115, may be used to corroborate the identity of the individual returning the medication to the dispensing cabinet 130.

Alternatively and/or additionally, the surveillance system 135 may be used to count a number of medications, or a quantity of a medication in a dose (e.g., a number of pills) retrieved from a drawer of the dispensing cabinet 130. For example, the detection engine 115 may determine, based on the recordings from at least one of the cameras (e.g., the second camera 210B, the third camera 210C, the fourth camera 210D, and the fifth camera 210E) pointing at the corresponding drawers, the quantity of medication retrieved from the particular drawer. The diversion controller 110 may determine based on the recordings, whether the correct amount of the medication has been withdrawn from the drawers of the dispensing cabinet 130.

For example, via the input/output device 230, the diversion controller 110 may request that the clinician show the quantity of the medication withdrawn from the drawers, scan a barcode or other identifier, representing the medication type or other information about the medication, at the input/output device 230, and/or the like. Based on the captured recordings, the detection engine 115 may record the detected quantity, and compare the detected quantity to an expected quantity based on one or more user inputs. Based on the comparison, the detection engine 115 may determine that a possible diversion event has occurred when the detected quantity of medication does not correspond to the expected quantity. In some embodiments, upon the detection of a diversion event or a possible diversion event, the detection engine 115 may generate one or more alerts. Such configurations may help to improve diversion detection because the quantity of a medication actually withdrawn may be more easily and accurately compared to the quantity of medication that should have been withdrawn or wasted by the clinician. Such configurations may also help to detect if the incorrect medication has been withdrawn and/or if an expired medication has been withdrawn (e.g., via the barcode scanning).

The configurations may be established for users of the system. A configuration may identify which sensors or input devices to activate. A configuration may include identification of what information is required to be collected by the activated sensors or input devices. For example, if a camera is identified for activation, a number of images to capture may be identified by the configuration. In some implementations, the configuration may identify an analysis engine for determining the sufficiency of the collected information. For example, if a camera is identified for activation, an image recognition engine may be identified to determine whether the image identifies the desired elements for an adequate evidence record (e.g., facial recognition, medication recognition, optical character recognition, or others). As another example, if an audio capture device is identified, the analysis may include volume detection, voice recognition, keyword or speech recognition, or the like.

A set of configurations may be defined to dynamically select an appropriate configuration. The selection may be based on substance type (e.g., controlled substance transactions), user or user classification (e.g., specific users, users with a threshold risk score, "junior" nursing staff, traveling nurses, etc.), by time of the witnessing session (e.g., day, night, day of week, holidays, etc.), location of witnessing (e.g., if a user is accessing medications outside of their assigned care area), or other detectable characteristic of the user or dispensing transaction. The system may include one or more graphical user interfaces to facilitate definition of configurations by offering control elements to receive and verify the relevant parameters for configurations.

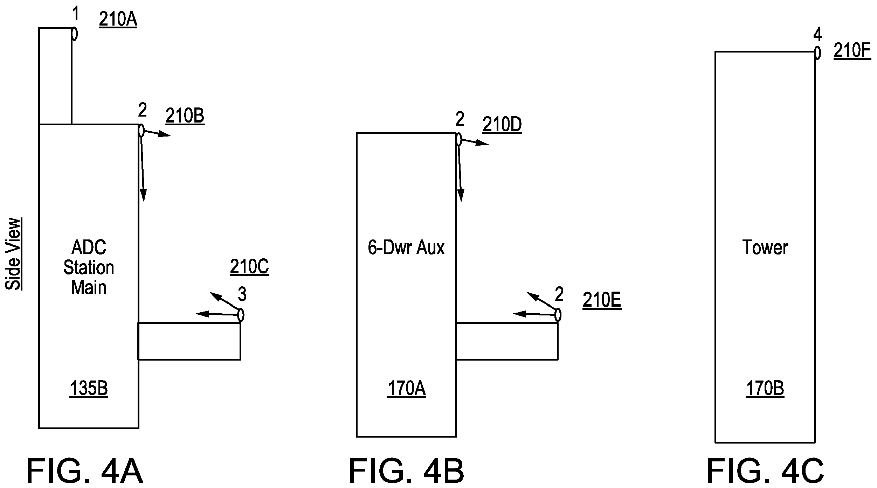

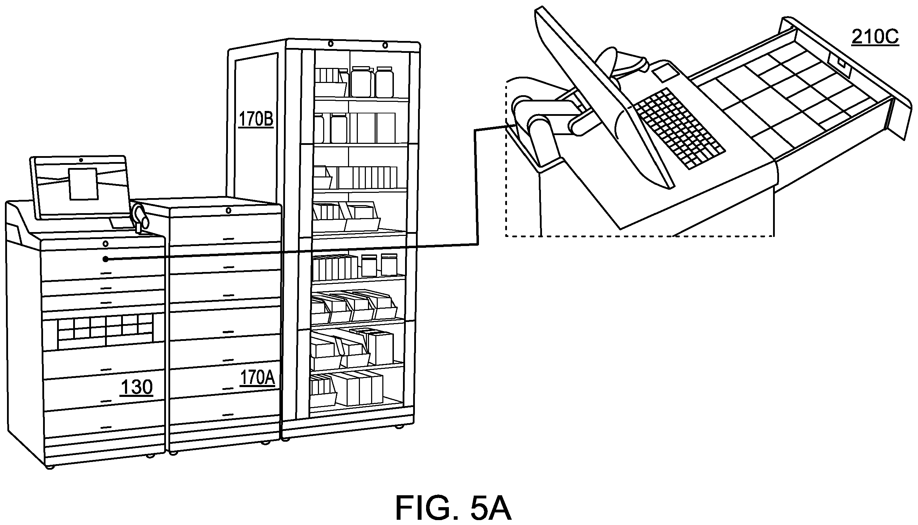

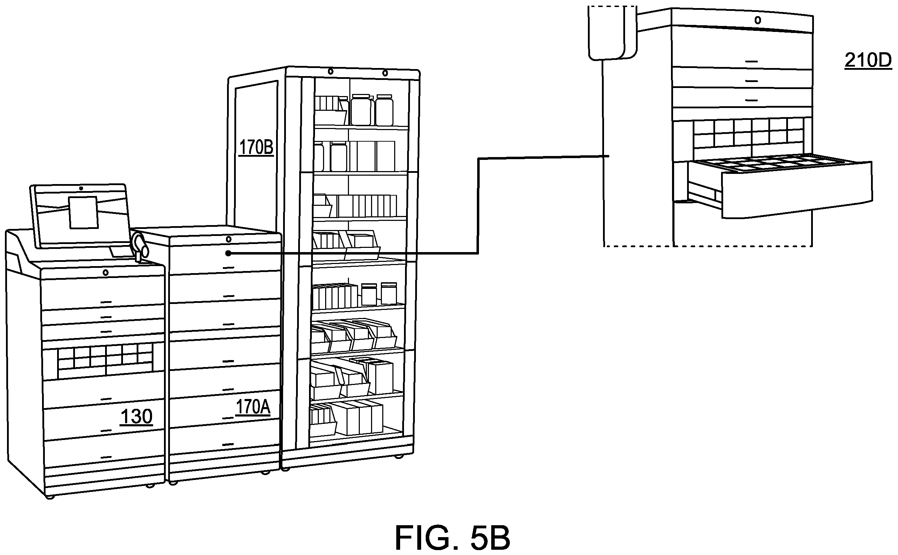

FIGS. 4A-4C illustrate side views of the dispensing cabinet 130 and the first and second auxiliary devices 170A, 170B, which may be coupled (e.g., via a wired or wireless connection) to the dispensing cabinet 130. As shown, one or more surveillance devices may be positioned at various locations. For example, on the dispensing cabinet 130, a first surveillance device (e.g., the first camera 210A) may be positioned to capture the clinician's face and/or a keyboard of the input/output device 230 (also see FIG. 5A), a second camera (e.g., the second camera 210B) may be positioned to capture the clinician moving the drawer of the dispensing cabinet 130 and/or withdrawing medication from the dispensing cabinet 130, and a third camera (e.g., the third camera 210C) may be positioned to capture the interior of the drawers. As shown in FIG. 4B, the first auxiliary device 170A may include a fourth camera (e.g., the fourth camera 210D) that may be positioned to capture the clinician moving the drawer of the dispensing cabinet 130 and/or withdrawing medication from a drawer of the first auxiliary device 170A (see also FIG. 5B), and a fifth camera (e.g., the fifth camera 210E) that may be positioned to capture the interior of the drawers. In some embodiments, the second auxiliary device 170B also includes a sixth camera (e.g., a sixth camera 210F) that captures one or more regions of the room of the patient.

Referring again to FIG. 1, the one or more data systems 160 may include an inventory system 160A, a patient care system 160B, an administrative system 160C, and an electronic medical record (EMR) system 160D. In some embodiments, a triggering event may occur, causing the surveillance system 135 to begin an investigative workflow (e.g., record, via a surveillance device, a video, a still image, audio, and/or the like), generate one or more transaction records corresponding to the recordings, generate an alert, and/or the like. A triggering event may occur when a clinician (or other individual) enters a patient room, is within a predetermined distance (e.g., one, two, three, four, or five or more feet) from the dispensing cabinet 130 for a threshold amount of time (e.g., 30 seconds, 1 minute, 5 minutes, 30 minutes, 1 hour, or more), attempts to access or accesses the dispensing cabinet 130, and/or interacts with the identification device 147 of the dispensing cabinet 130. The triggering event may additionally and/or alternatively occur when one or more of the surveillance devices are at least partially occluded and/or become dark, and/or the like. The triggering event may additionally and/or alternatively occur when the diversion detection system detects that an individual who has been previously identified as a possible diverter, enters the room of the patient.

For example, a clinician dispensing a medication for a patient from the dispensing cabinet 130 may cause the surveillance system 135 to begin the investigative workflow, and the diversion controller 110 to generate a transaction record that corresponds to the investigative workflow. Each transaction record may include one or more transaction values corresponding to, for example, a timestamp indicating the start and/or end time of the transaction, a clinician identifier of the clinician, a device identifier of the dispensing cabinet and/or auxiliary device, a patient identifier of the patient, a medication identifier of the medication retrieved from the dispensing cabinet, a quantity of the medication retrieved from the medication cabinet, prescription order identifiers, inventory information, patient status, shift identifiers, location tracking identifiers, electronic health record identifiers, a transaction type, a witness identifier, and/or the like. Alternatively and/or additionally, a clinician using a wasting station to waste unused medication from the patient may trigger the generation of another transaction record that includes a timestamp, the clinician identifier of the clinician, a clinician identifier of another clinician witnessing the wasting, a device identifier of the wasting station, the patient identifier of the patient, an identifier of the medication being wasted, a quantity of the medication being wasted, and/or the like. As used herein, the "wasting" of a medication may refer to the disposal of a substance in accordance with institutional guidelines and/or government regulations. For example, the proper wasting of a prescription pain medication may require the controlled substance to be collected in a designated receptacle (e.g., wasting stations) while in the presence of one or more witnesses.

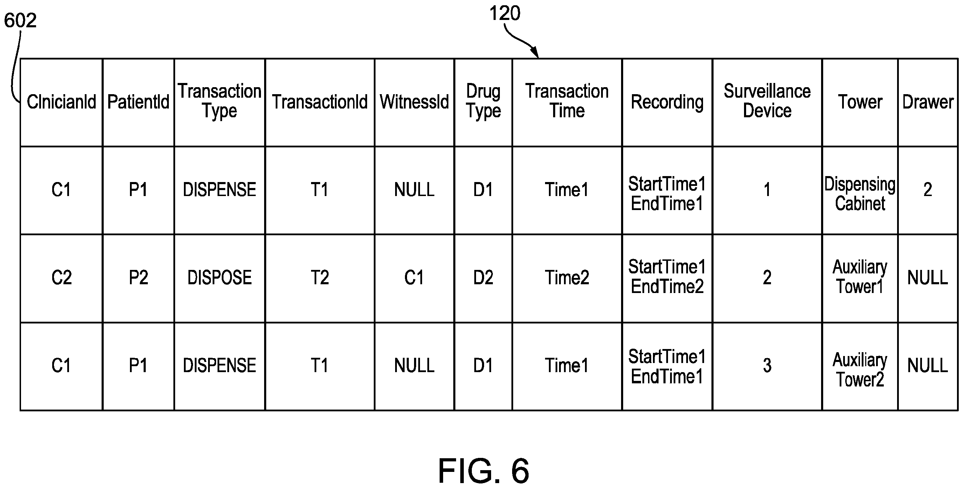

The diversion controller 110 may generate the transaction records at the time the triggering event is detected, at predetermined time intervals during a triggering event (e.g., every 30 seconds, 1 minute, 30 minutes, 1 hour, 12 hours, etc.) after a triggering event has been detected, and/or continuously after a triggering event has been detected. The transaction records may linked to a recording of the surveillance system 135 and be transmitted to and/or stored locally at the dispensing cabinet 130. In some embodiments, the transaction records may be linked to a recording of the surveillance system 135 and transmitted to a database to be later accessed and/or filtered to detect that a diversion has taken place, identify a possible diverter, and/or the like. FIG. 6 illustrates an example of the transaction records 602 generated by the diversion controller 110 corresponding to one or more recordings that are stored at the dispensing cabinet 130 and/or at the database 120 of the diversion controller 110.

Additionally, and/or alternatively, in response to the detection of a triggering event, such as when one or more of the surveillance devices of the surveillance system 135 are at least partially occluded, the diversion controller 110 may trigger an alert, which may include a notification that may be provided via a user interface 145 at the client 140. For example, the notification may be provided via a short messaging service (SMS) text, an email, a webpage, an application, and/or the like. Additionally and/or alternatively, in response to the detection of a triggering event, the diversion controller 10 may cause an increase in a sampling rate of one or more of the surveillance devices of the surveillance system 135, record video and/or images in color, rather than in black and white, divert additional electronic and/or memory resources to the surveillance system 135 and/or the like.

At least a portion of the data (e.g., the transaction records) captured and linked to a recording of the surveillance system 135, for example, by the diversion controller 110, the surveillance system 135 and/or the identification device 147, together with the corresponding recordings, may be stored at the dispensing cabinet 130, and/or may be transmitted to the diversion controller 110 (or a database 120 coupled with the diversion controller 110) via the network 150.

Alternatively and/or additionally, the diversion controller 110 may also receive, via the network 150, data from the one or more data systems 160, such as the inventory system 160A, the patient care system 160B, the administrative system 160C, the EMR system 160D, and/or the like. It should be appreciated that the one or more data systems 160 may also provide non-transactional data. For instance, the EMR system 160D may store a plurality of electronic medical records (EMRs), each of which including a patient's history including, for example, past opioid use and/or the like. While the clinician interacting with the EMR system 160D to create, update, and/or retrieve an EMR for the patient may generate a timestamp transaction record, the electronic medical record itself may not be a transaction record. Similarly, it should be appreciated that the diversion controller 110 may be coupled with any number of computing systems capable of providing data associated with the flow of medication throughout one or more medical facilities including, for example, from being delivered and inducted into the inventory system 160A to being dispensed from the dispensing cabinet 130, administered to patients via the patient care system 160B, and/or ultimately wasted by being disposed of at the dispensing cabinet 130. For example, the inventory system 160A may be configured to track the supply of medication available at one or more medical facilities. The patient care system 160B may include one or more devices for administering medications to patients including, for example, patient controlled analgesic pumps. The administrative system 160C may track the personnel at one or more medical facilities including, for example, personal data, shift schedules, vacation days, and/or the like.

In some embodiments, the inventory system 160A, the patient care system 160B, the administrative system 160C, and/or the EMR system 160D may provide electronic medication administration records including, for example, patient records, caregiver records, medication records, and/or the like. The patient records may include, for example, patient identifications, patient locations, demographics, health data (e.g., pain scales, vitals, and/or the like), caregiver assignments, and/or the like. The caregiver records may include, for example, caregiver identifications, caregiver schedules and/or actual times worked, caregiver locations, caregiver patient assignments, and/or the like. The medication records may include, for example, date and/or time of medication administration, identification of administered medications, strength and/or concentration of administered medications, dosage of administered medications, dosage forms of administered medications, date and/or time of medication orders, strength and/or concentration of ordered medications, dosage of ordered medications, dosage form of ordered medications, and/or the like.

The data (e.g., the captured recordings, the transaction records, and the non-transactional records) received at the diversion controller 110 may be evaluated by the diversion controller 110 in real time and/or stored at a database 120 coupled with the diversion controller 110 for evaluation at a later time. In some example embodiments, the diversion controller 110 may include a machine learning engine configured to detect, based on the data received at the diversion controller 110 and/or stored at the database 120, diversion of medication, suspicious behavior indicative of the diversion of medication, and/or the like and flag the diversion or suspicious behavior. The diversion controller 110 may additionally and/or alternatively include a detection engine 115 configured to detect diversion or possible diversion of medication by generating the transaction records, linking the transaction record to a corresponding recording, filtering the transaction records based on one or more of the related transaction records, and/or triggering one or more alerts based on the filtered transaction records.

In some embodiments, via the detection engine 115, the diversion detection system 100 may detect or otherwise identify anomalous behavior indicating that a diversion has or may have occurred. Anomalous behavior may include the triggering event described above. In some embodiments, anomalous behavior may include accessing the dispensing cabinet at inconsistent times and/or with abnormal frequency, irregularities in wasted and/or returned medications, routinely selecting the same clinician to witness the wasting of medications, tailgating in which a clinician routinely accesses a medication management device within a threshold quantity of time after another clinician, withdrawal of medication for a discharged and/or deceased patient, excessive cancellations and/or overrides, and/or the like. For example, one caregiver assigned to a patient may access the dispensing cabinet at inconsistent times, with abnormal frequency relative to other caregivers assigned to the same patient and/or at times inconsistent with that individual's scheduled shifts (e.g., as indicated by data from the administrative system 160C). As another example, the caregiver may dispense and document the administration of a higher total dose of medication during their shift than the other caregivers, even when the patient does not report a change in pain. Alternatively and/or additionally, the patient may report inadequate pain relief even though the caregiver administers the same total dose of medication to the patient as the other caregivers assigned to the patient. Abnormally long periods of time may also elapse between when the caregiver dispenses the medication from the dispensing cabinet 130, administers the medication to the patient, and returns the unused medication to the dispensing cabinet 130 for disposal.

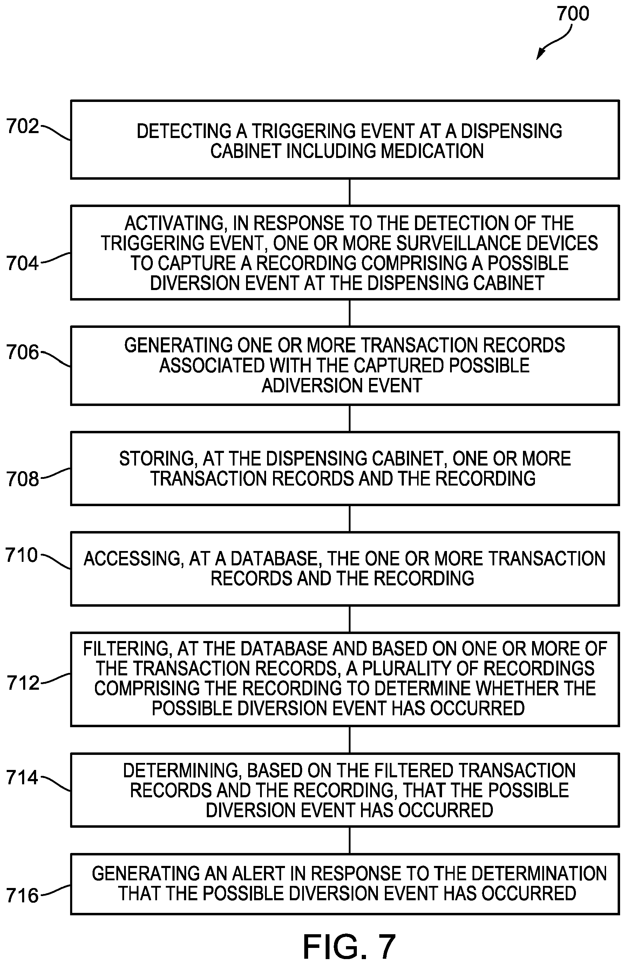

FIG. 7 depicts a flowchart illustrating a process 700 for generating evidence, by a diversion detection system (e.g., the diversion detection system 100) to identify a possible diversion event, during which medication may be diverted, in accordance with some example embodiments. A possible diversion event may occur at any point in time during the compounding, dispensing, administration, and/or wasting of the medication. For example, the diversion event may take many forms, including the removal of more doses than are permitted to be dispensed, removal of a dose and replacement with an illegitimate dose, replacement of a liquid controlled substance with saline or other liquids, removal of the incorrect type of medication and/or the like. The diversion detection system may include at least one dispensing cabinet including a medication. The diversion detection system may additionally and/or alternatively include a surveillance device having an area of detection that includes the at least one dispensing cabinet.

At 702, a diversion controller (e.g., the diversion controller 110) may detect a triggering event at a dispensing cabinet (e.g., the dispensing cabinet 130) including medication. The dispensing cabinet may be coupled to the diversion controller via a wired and/or wireless connection. In some embodiments, the triggering event may include a clinician (or other individual) entering a patient room, an individual being positioned within a predetermined proximity (e.g., one, two, three, four, or five or more feet) of the dispensing cabinet for a threshold amount of time (e.g., 30 seconds, 1 minute, 5 minutes, 30 minutes, 1 hour, or more), which may be detected by a motion detection device coupled with the dispensing cabinet, an individual accessing or attempting to access the dispensing cabinet, and/or an individual otherwise interacting with the identification device (e.g., the identification device 147) of the dispensing cabinet or another part of the dispensing cabinet (or auxiliary devices), such as the drawers of the dispensing cabinet and/or the input/output device coupled with the dispensing cabinet. The triggering event may additionally and/or alternatively include detecting that one or more of the surveillance devices are at least partially occluded, are damaged, and/or the like. The triggering event may additionally and/or alternatively include detecting that an individual who has been previously identified as a possible diverter, enters the room of the patient and/or attempts to access the dispensing cabinet.

At 704, the diversion controller may activate, in response to the detection of the triggering event, one or more surveillance devices to capture a recording. The recording may include a possible diversion event that occurred at the dispensing cabinet. The one or more surveillance devices (e.g., the one or more surveillance devices from the surveillance system 135) may include any recording device including, for example, a video camera, a still image camera, an audio recorder, and/or the like. The surveillance devices may be coupled to, be positioned on, or otherwise form a part of the dispensing cabinet, an auxiliary device, such as an auxiliary device coupled to the dispensing cabinet, and/or a room of the patient. The surveillance devices may be configured to capture images, videos, and/or audio recordings of individuals accessing the dispensing cabinet, for example, to retrieve and/or return medication.

In some embodiments, upon detection of the triggering event, the surveillance system, for instance, may move one or more surveillance devices, change the focus and/or zoom of one or more surveillance devices, and/or activate additional surveillance devices (such as at the dispensing cabinet, within a drawer of the dispensing cabinet, at the auxiliary device, and/or at other locations) in order to capture images and/or videos of the individual at specific angles such as, for example, overhead shots of the individual's hands manipulating medication within the dispensing cabinet. The activation at 704 may be dynamic based on the configuration applicable to the transaction. The activation at 704 may include collecting the necessary criteria to select an appropriate configuration from a set of configurations.

At 706, one or more transaction records may be generated by the diversion controller. In some implementations, the one or more transaction records may be linked to at least a portion of the recording that comprises the possible diversion event. For example, the transaction records may be associated with the captured possible diversion event in the recording. Each of the transaction records may include one or more transaction values corresponding to, for example, a timestamp indicating the start and/or end time of the transaction, a clinician identifier of the clinician, a device identifier of the dispensing cabinet and/or auxiliary device, a surveillance device identifier of the surveillance devices (e.g., positioned on or in the dispensing cabinet, auxiliary device, or other location), a patient identifier of the patient, a medication identifier of the medication retrieved from the dispensing cabinet, a quantity of the medication retrieved from the medication cabinet, prescription order identifiers, inventory information, patient status, shift identifiers, location tracking identifiers, electronic health record identifiers, a transaction type, a witness identifier, and/or the like. Alternatively and/or additionally, a clinician using a wasting station to waste unused medication from the patient may trigger the generation of another transaction record that includes a timestamp, the clinician identifier of the clinician, a clinician identifier of another clinician witnessing the wasting, a device identifier of the wasting station, the patient identifier of the patient, an identifier of the medication being wasted, a quantity of the medication being wasted, and/or the like. Accordingly, the transaction records may include data associated with one or more individuals accessing the dispensing cabinet to retrieve and/or return the medication.

Additionally and/or alternatively, the surveillance system and/or the diversion controller may be configured to capture transaction records associated with individuals accessing the dispensing cabinet, the auxiliary device and/or a patient care room, for example, to retrieve medication from the dispensing cabinet and/or the auxiliary device, and/or return unused medication to the dispensing cabinet and/or the auxiliary device. Alternatively and/or additionally, the surveillance system may capture transaction records during, for example, logins to and/or logouts from the dispensing cabinet and/or the auxiliary device, searches for patients and/or medications at the dispensing cabinet and/or the auxiliary device, patient record reviews, counting and documenting the count of medications in the dispensing cabinet and/or the auxiliary device, fill and refills of medications, loading and/or unloading medication from the dispensing cabinet and/or the auxiliary device, documentation of discrepancies, cancellation of transactions (e.g., dispense, fill/refill, load/unload, count, waste, return, and/or the like), and/or the like.

As noted above, generating the one or more transaction records may also include linking the one or more transaction records with at least a portion of the recording that includes the captured possible diversion event. For example, the one or more transaction records may be linked to all or a portion of a recording, by storing the transaction record in association with a timestamp indicating the start and/or end time of the triggering event (e.g., the transaction), a device identifier identifying the device to which the surveillance device that captured the recording is coupled (e.g., the dispensing cabinet and/or auxiliary device), a surveillance device identifier of the surveillance device (e.g., positioned on or in the dispensing cabinet, auxiliary device, or other location) that captured the relevant portion of the recording, a clinician identifier identifying the clinician that accessed the dispensing cabinet, a medication identifier identifying the medication withdrawn from and/or wasted at the dispensing cabinet, and/or the like. Linking the one or more transaction records with at least a portion of the recording that includes the captured possible diversion event allows for each of the portions of the recording to be filtered more easily by the associated transaction records. This may reduce the required device resources (e.g., power, processing time, memory, network bandwidth, and the like) to determine whether a diversion event occurred. For example, such configurations may help to more quickly sort the transaction records and/or a relevant portion of the recording, to quickly and/or efficiently determine whether a diversion occurred and if the diversion event occurred, the individual, medication, and/or patient associated with the diversion event. Accordingly, linking the transaction records with at least the portion of the recording that includes the possible diversion event may significantly reduce the amount of time it takes to identify that a diversion has occurred and the clinician or patient involved with the diversion, prevent the diversion event from occurring or continuing, and/or reduce the likelihood that the diversion will occur.

In some embodiments, the generated transaction records and at least a portion of the linked recording are transmitted to a main surveillance device hub at the dispensing cabinet. Thus, at 708, the one or more transaction records and the recording captured by the one or more surveillance devices may be stored at the dispensing cabinet. For example, the generated transaction records and at least a portion of the linked recording may be transmitted directly from one or more of the surveillance devices. As another example, the generated transaction records and at least a portion of the linked recording may be transmitted first to an auxiliary surveillance device hub located at one or more of the auxiliary devices, and then transmitted to the main surveillance device hub at the dispensing cabinet. In other words, the dispensing cabinet (e.g., via the main surveillance device hub) may receive and/or store (e.g., temporarily) the generated transaction records and at least a portion of the linked recording.

At 710, the one or more transaction records and the associated recording captured by the one or more surveillance devices may be accessed at a database coupled with the diversion controller. In some embodiments, the generated transaction records and at least a portion of the linked recording may be transmitted to a database coupled with the diversion controller. For example, the generated transaction records and at least a portion of the linked recording may be transmitted directly from the one or more surveillance devices, the auxiliary surveillance device hub, and/or from the main surveillance device hub. Accordingly, in some embodiments, the generated transaction records and at least a portion of the linked recording may be first transmitted to the dispensing cabinet and then to the database coupled with the diversion controller. The database coupled with the diversion controller may be located remotely relative to the main surveillance device hub and/or the dispensing cabinet.

At 712, a plurality of recordings comprising the recording may be filtered, at the database and based on one or more of the transaction records, to determine whether the possible diversion event has occurred. For example, one or more transaction records may be selected. Based on the selection of the one or more transaction records, the diversion controller may access the database coupled with the diversion controller, and display, via a user input/output coupled with the diversion controller, such as a display that is remote from the dispensing cabinet, the at least a portion of the recording that is linked to the selected transaction records.

At 714, the diversion controller may detect, based on the filtered transaction records and the recording, that the possible diversion event has occurred. In some example embodiments, the diversion controller may detect, based on the transaction records and/or the recording, that the possible diversion event has occurred based on suspicious or anomalous behavior indicative of the diversion of medication, and/or the like, and flag the diversion or behavior. For example, the diversion controller may determine that the individual associated with the possible diversion event (which may be detected based on the transaction records linked with the recording) has exhibited an anomalous behavior.

Anomalous behavior may include the triggering event described above. In some embodiments, anomalous behavior may include accessing the dispensing cabinet at inconsistent times and/or with abnormal frequency, irregularities in wasted and/or returned medications, routinely selecting the same clinician to witness the wasting of medications, tailgating in which a clinician routinely accesses a medication management device within a threshold quantity of time after another clinician, withdrawal of medication for a discharged and/or deceased patient, excessive cancellations and/or overrides, and/or the like. In some embodiments, anomalous behavior may include determining that one or more of the surveillance devices has been blocked or at least partially occluded. For example, one or more sensors (e.g., light sensors, motion sensors, and the like) may determine that one or more of the surveillance devices has been blocked or at least partially occluded.

At 716, the diversion controller may, in response to the determination that the possible diversion event has occurred, generate an alert indicating that the possible diversion event has occurred and/or may have occurred. An alert may include a notification that may be provided via a user interface at the client, the dispensing cabinet, and/or a remote display. For example, the notification may be provided via a short messaging service (SMS) text, an email, a webpage, an application, and/or the like. Additionally, and/or alternatively, in response to the detection of an initial triggering event, such as when one or more of the surveillance devices of the surveillance system are at least partially occluded, the diversion controller may trigger an alert.

Additionally, and/or alternatively, the diversion controller may, in response to the determination that the possible diversion event has occurred, transmit a message to control the dispensing cabinet or other hardware within the environment. For example, a control message may be transmitted to lock the dispensing cabinet or otherwise prevent access to one or more drawers of the dispensing cabinet. Additionally and/or alternatively, the diversion controller may, in response to the determination that the possible diversion event has occurred, request an additional witness, additional evidence collection, or other authorization before permitting access to the dispensing cabinet. Accordingly, the diversion detection system described herein may desirably generate useful and sortable evidence of the diversion of medication that may help to more easily and quickly identify the individual responsible for the diversion.

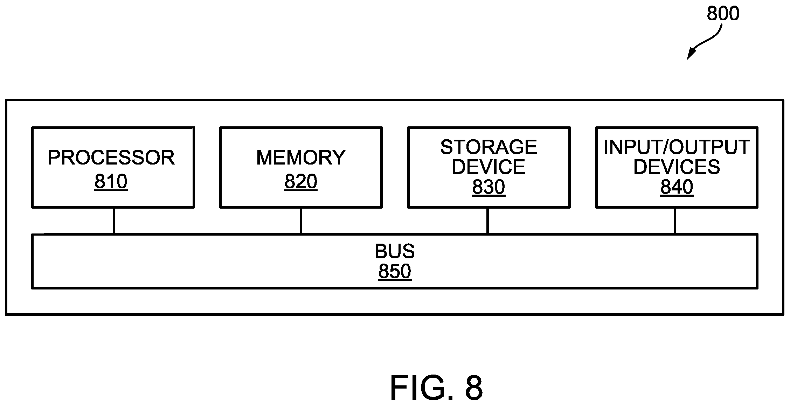

As shown in FIG. 8, the computing system 800 can include a processor 810, a memory 820, a storage device 830, and input/output devices 840. The processor 810, the memory 820, the storage device 830, and the input/output devices 840 can be interconnected via a system bus 850. The processor 810 is capable of processing instructions for execution within the computing system 800. In some example embodiments, the processor 810 can be a single-threaded processor. Alternatively, the processor 810 can be a multi-threaded processor. The processor 810 is capable of processing instructions stored in the memory 820 and/or on the storage device 830 to present graphical information for a user interface provided via the input/output device 840.

The memory 820 is a computer readable medium such as volatile or nonvolatile that stores information within the computing system 800. The memory 820 can store data structures representing configuration object databases, for example. The storage device 830 is capable of providing persistent storage for the computing system 800. The storage device 830 can be a floppy disk device, a hard disk device, an optical disk device, or a tape device, or other suitable persistent storage means. The input/output device 840 provides input/output operations for the computing system 800. In some example embodiments, the input/output device 840 includes a keyboard and/or pointing device. In various embodiments, the input/output device 840 includes a display unit for displaying graphical user interfaces.

According to some example embodiments, the input/output device 840 can provide input/output operations for a network device. For example, the input/output device 840 can include Ethernet ports or other networking ports to communicate with one or more wired and/or wireless networks (e.g., a local area network (LAN), a wide area network (WAN), the Internet).

In some example embodiments, the computing system 800 can be used to execute various interactive computer software applications that can be used for organization, analysis and/or storage of data in various formats. Alternatively, the computing system 800 can be used to execute software applications. These applications can be used to perform various functionalities, e.g., planning functionalities (e.g., generating, managing, editing of spreadsheet documents, word processing documents, and/or any other obj ects, etc.), computing functionalities, communications functionalities, etc. The applications can include various add-in functionalities or can be standalone computing products and/or functionalities. Upon activation within the applications, the functionalities can be used to generate the user interface provided via the input/output device 840. The user interface can be generated and presented to a user by the computing system 800 (e.g., on a computer screen monitor, etc.).

Several embodiments discuss the evidence generation in relation to diversion detection. The features may be included to additionally or alternatively confirm compliance. For example, aspects of the system may be provided within a school to dispense medications to specific students. The features described facilitate generation of evidence to indicate which student took which medication at a specific location and time. The guided collection of the relevant data points allows even novice (e.g., children) to efficiently and safely receive the appropriate medications.

One or more aspects or features of the subject matter described herein can be realized in digital electronic circuitry, integrated circuitry, specially designed ASICs, field programmable gate arrays (FPGAs) computer hardware, firmware, software, and/or combinations thereof. These various aspects or features can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device. The programmable system or computing system may include clients and servers. A client and server are remote from each other and typically interact through a communication network. The relationship of client and server arises by virtue of computer programs running on the respective computers and having a client-server relationship to each other.

These computer programs, which can also be referred to as programs, software, software applications, applications, components, or code, include machine instructions for a programmable processor, and can be implemented in a high-level procedural and/or obj ect-oriented programming language, and/or in assembly/machine language. As used herein, the term "machine-readable medium" refers to any computer program product, apparatus and/or device, such as for example magnetic discs, optical disks, memory, and Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor. The machine-readable medium can store such machine instructions non-transitorily, such as for example as would a non-transient solid-state memory or a magnetic hard drive or any equivalent storage medium. The machine-readable medium can alternatively or additionally store such machine instructions in a transient manner, such as for example, as would a processor cache or other random access memory associated with one or more physical processor cores.

To provide for interaction with a user, one or more aspects or features of the subject matter described herein can be implemented on a computer having a display device, such as for example a cathode ray tube (CRT) or a liquid crystal display (LCD) or a light emitting diode (LED) monitor for displaying information to the user and a keyboard and a pointing device, such as for example a mouse or a trackball, by which the user may provide input to the computer. Other kinds of devices can be used to provide for interaction with a user as well. For example, feedback provided to the user can be any form of sensory feedback, such as for example visual feedback, auditory feedback, or tactile feedback; and input from the user may be received in any form, including acoustic, speech, or tactile input. Other possible input devices include touch screens or other touch-sensitive devices such as single or multi-point resistive or capacitive track pads, voice recognition hardware and software, optical scanners, optical pointers, digital image capture devices and associated interpretation software, and the like.

In the descriptions above and in the claims, phrases such as "at least one of" or "one or more of" may occur followed by a conjunctive list of elements or features. The term "and/or" may also occur in a list of two or more elements or features. Unless otherwise implicitly or explicitly contradicted by the context in which it used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." Use of the term "based on," above and in the claims is intended to mean, "based at least in part on," such that an unrecited feature or element is also permissible.

As used herein a "user interface" (also referred to as an interactive user interface, a graphical user interface or a UI) may refer to a network based interface including data fields and/or other control elements for receiving input signals or providing electronic information and/or for providing information to the user in response to any received input signals. Control elements may include dials, buttons, icons, selectable areas, or other perceivable indicia presented via the UI that, when interacted with (e.g., clicked, touched, selected, etc.), initiates an exchange of data for the device presenting the UI. A UI may be implemented in whole or in part using technologies such as hyper-text mark-up language (HTML), FLASH^{™}, JAVA^{™}, .NET^{™}, web services, or rich site summary (RSS). In some embodiments, a UI may be included in a stand-alone client (for example, thick client, fat client) configured to communicate (e.g., send or receive data) in accordance with one or more of the aspects described. The communication may be to or from a medical device or server in communication therewith.

As used herein, the terms "determine" or "determining" encompass a wide variety of actions. For example, "determining" may include calculating, computing, processing, deriving, generating, obtaining, looking up (e.g., looking up in a table, a database or another data structure), ascertaining and the like via a hardware element without user intervention. Also, "determining" may include receiving (e.g., receiving information), accessing (e.g., accessing data in a memory) and the like via a hardware element without user intervention. "Determining" may include resolving, selecting, choosing, establishing, and the like via a hardware element without user intervention.

As used herein, the terms "provide" or "providing" encompass a wide variety of actions. For example, "providing" may include storing a value in a location of a storage device for subsequent retrieval, transmitting a value directly to the recipient via at least one wired or wireless communication medium, transmitting or storing a reference to a value, and the like. "Providing" may also include encoding, decoding, encrypting, decrypting, validating, verifying, and the like via a hardware element.

As used herein, the term "message" encompasses a wide variety of formats for communicating (e.g., transmitting or receiving) information. A message may include a machine readable aggregation of information such as an XML document, fixed field message, comma separated message, or the like. A message may, in some embodiments, include a signal utilized to transmit one or more representations of the information. While recited in the singular, it will be understood that a message may be composed, transmitted, stored, received, etc. in multiple parts.

As user herein, the terms "correspond" or "corresponding" encompasses a structural, functional, quantitative and/or qualitative correlation or relationship between two or more objects, data sets, information and/or the like, preferably where the correspondence or relationship may be used to translate one or more of the two or more objects, data sets, information and/or the like so to appear to be the same or equal. Correspondence may be assessed using one or more of a threshold, a value range, fuzzy logic, pattern matching, a machine learning assessment model, or combinations thereof.

In any embodiment, data generated or detected can be forwarded to a "remote" device or location, where "remote," means a location or device other than the location or device at which the program is executed. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet or including email transmissions and information recorded on websites and the like.

As used herein, the terms "control" or "controlling" encompass a wide variety of actions. For example, "controlling" a device may include transmitting one or more messages to adjust an operational state or functional element of the device. The message may include specific instructions to be executed by a processor of the device to manifest the change. The "controlling" may include storing a value in a location of a storage device for subsequent retrieval by the device to be controlled, transmitting a value directly to the device to be controlled via at least one wired or wireless communication medium, transmitting or storing a reference to a value, and the like. For example, a control message may include a value to adjust a level of power from a power source of the controlled device. As another example, a control message may activate or deactivate a structural element of the controlled device such as a light, audio playback, a motor, a lock, a pump, a display, a servo, or other component of a device described herein. "Controlling" may include indirect control of the device by adjusting a configuration value used by the controlled device. For example, the control message may include a threshold value for a device characteristic (e.g., temperature, rate, frequency, etc.). The threshold value may be stored in a memory location and referred to by the controlled device during operation.

The subject matter described herein can be embodied in systems, apparatus, methods, and/or articles depending on the desired configuration. The embodiments set forth in the foregoing description do not represent all embodiments consistent with the subject matter described herein. Instead, they are merely some examples consistent with aspects related to the described subject matter. Although a few variations have been described in detail above, other modifications or additions are possible. In particular, further features and/or variations can be provided in addition to those set forth herein. For example, the embodiments described above can be directed to various combinations and subcombinations of the disclosed features and/or combinations and subcombinations of several further features disclosed above. In addition, the logic flows depicted in the accompanying figures and/or described herein do not necessarily require the particular order shown, or sequential order, to achieve desirable results. Other embodiments may be within the scope of the following claims.

## Claims

1. A system for automated dispensing of medications, comprising:
at least one dispensing cabinet (130) including a medication;
a surveillance device (135A, 135B) having an area of detection that includes the at least one dispensing cabinet (130);
at least one data processor (810); and
at least one memory (820) storing instructions which, when executed by the at least one data processor (810), result in operations comprising:
detecting a triggering event at the at least one dispensing cabinet (130);
activating, in response to the detection of the triggering event, one or more surveillance devices (135A, 135B) to capture a recording comprising a possible diversion event at the at least one dispensing cabinet (130), wherein the one or more surveillance devices (135A, 135B) comprises the surveillance device (135A, 135B);
generating, based on the triggering event, one or more transaction records (602) associated with the recording, wherein the one or more transaction records (602) comprise one or more of a clinician identifier, a patient identifier, a transaction type, a transaction identifier, a witness identifier, and a medication type;
linking the one or more transaction records (602) to at least a portion of the recording that comprises the captured possible diversion event, wherein the one or more transaction records (602) are linked to at least the portion of the recording using at least a timestamp indicating a start time and an end time of the triggering event;
filtering a plurality of recordings including the recording comprising the possible diversion event to identify one or more recordings comprising the possible diversion event, wherein the filtering is based on the one or more transaction records (602) linked to the at least a portion of the recording that comprises the captured possible diversion event;
determining the possible diversion event occurred based on the identified one or more recording comprising the possible diversion event and the one or more transaction records (602) linked to the at least a portion of the recording that comprises the captured possible diversion event; and
in response to a determination that the possible diversion event occurred, generating an alert indicating that the possible diversion event has occurred.

2. The system of claim 1, wherein the one or more transaction records (602) are further linked to at least the portion of the recording using
a surveillance device identifier of a surveillance device (135A, 135B) of the one or more surveillance devices (135A, 135B) that captured at least the portion of the recording.

3. The system of any of claims 1 or 2, wherein the operations further comprise: storing, at the at least one dispensing cabinet (130), the linked one or more transaction records (602) and at least the portion of the recording.

4. The system of any of claims 1 to 3, wherein the plurality of recordings are filtered at a database (120) remote from the at least one dispensing cabinet (130).

5. The system of any of claims 1 to 4, wherein the operations further comprise: accessing, at a database (120) remote from the at least one dispensing cabinet (130), the one or more transaction records (602) and the recording captured by the one or more surveillance devices (135A, 135B).

6. The system of any of claims 1 to 5, wherein the operations further comprise: determining, based on filtered transaction records and the recording that the possible diversion event has occurred.

7. The system of any of claims 1 to 6, wherein the triggering event comprises one or more of: an individual entering a patient room, an individual being located within a predetermined proximity of the at least one dispensing cabinet (130) for a threshold amount of time, and an individual interacting with the at least one dispensing cabinet (130).

8. The system of any of claims 1 to 7, wherein the recording comprises at least one video, image, and audio recording.

9. The system of any of claims 1 to 8, wherein the surveillance device (135A, 135B) is positioned on the at least one dispensing cabinet (130); and wherein a second surveillance device (135A, 135B) of the one or more surveillance devices (135A, 135B) is positioned on an auxiliary device (170) coupled with the at least one dispensing cabinet (130).

10. The system of claim 9, wherein the captured possible diversion event is transmitted from an auxiliary surveillance device hub (137A, 137B) at the auxiliary device (170) to a main surveillance device hub (137) at the at least one dispensing cabinet (130).

11. The system of any of claims 1 to 10, wherein the operations further comprise:
detecting, based on the recording, a quantity of the medication retrieved from the at least one dispensing cabinet (130); wherein the determination that the possible diversion event occurred comprises determining that the detected quantity of the medication is different from an expected quantity of the medication.

12. The system of claim 1, wherein the operations further comprise: transmitting the linked transaction records (602) and at least the portion of the recording to a remote database (120) to enable the filtering at the database (120).

13. The system of claim 1, wherein detecting the triggering event causes the generation of the one or more transaction records (602).

14. The system of claim 1, wherein linking the one or more transaction records (602) to at least the portion of the recording that comprises the captured possible diversion event allows for each of the portions of the recording to be filtered more easily by the associated transaction records (602).

15. The system of claim 11, wherein the operations further comprise: requesting the quantity of the medication retrieved from the at least one dispensing cabinet (130) to be shown to at least the surveillance device (135A, 135B).

## Patentansprüche

1. Ein System zur automatischen Ausgabe von Medikamenten, umfassend:
mindestens ein Spenderschrank (130), der ein Medikament enthält;
eine Überwachungsvorrichtung (135A, 135B) aufweisend einen Erkennungsbereich, der den mindestens einen Ausgabeschrank (130) enthält;
mindestens einen Datenprozessor (810); und
mindestens einen Speicher (820), der Befehle speichert, die, wenn sie von dem mindestens einen Datenprozessor (810) ausgeführt werden, zu Operationen führen, welche umfassen:
Erkennen eines auslösenden Ereignisses an dem mindestens einen Ausgabeschrank (130);
Aktivieren einer oder mehrerer Überwachungsvorrichtungen (135A, 135B) als Reaktion auf die Erkennung des auslösenden Ereignisses, um eine Aufzeichnung zu erfassen, die ein mögliches Abzweigungsereignis an dem mindestens einen Ausgabeschrank (130) umfasst, wobei die eine oder mehreren Überwachungsvorrichtungen (135A, 135B) die Überwachungsvorrichtung (135A, 135B) umfasst;
Erzeugen, basierend auf dem auslösenden Ereignis, eines oder mehrerer Transaktionsdatensätze (602), die mit der Aufzeichnung verknüpft sind, wobei der eine oder mehreren Transaktionsdatensätze (602) einen oder mehrere von einem Kliniker-Identifikator, einem Patienten-Identifikator, einem Transaktionstyp, einem Transaktions-Identifikator, einem Zeugen-Identifikator und einem Medikationstyp umfasst;
Verknüpfen des einen oder mehreren Transaktionsdatensätze (602) mit mindestens einem Teil der Aufzeichnung, die das erfasste mögliche Abzweigungsereignis umfasst, wobei der eine oder mehrere Transaktionsdatensätze (602) mit mindestens dem Teil der Aufzeichnung unter Verwendung mindestens eines Zeitstempels verknüpft sind, der eine Startzeit und eine Endzeit des auslösenden Ereignisses angibt;
Filtern einer Vielzahl von Aufzeichnungen, welche die Aufzeichnung, die das mögliche Abzweigungsereignis umfasst, enthalten, um eine oder mehrere Aufzeichnungen zu identifizieren, die das mögliche Abzweigungsereignis umfassen, wobei das Filtern auf dem einen oder mehreren Transaktionsdatensätzen (602) basiert, die mit dem mindestens einen Teil der Aufzeichnung verbunden sind, der das erfasste mögliche Abzweigungsereignis umfasst;
Bestimmen, dass das möglichen Abzweigungsereignis eingetreten ist, basierend auf der identifizierten einen oder mehreren Aufzeichnung, die das mögliche Abzweigungsereignis und den einen oder mehrere Transaktionsdatensätze (602) umfasst, welche mit dem mindestens einen Teil der Aufzeichnung, die das erfasste mögliche Abzweigungsereignis umfasst, verknüpft sind; und
als Reaktion auf ein Bestimmen, dass das mögliche Abzweigungsereignis eingetreten ist, Erzeugen einer Warnung, die anzeigt, dass das mögliche Abzweigungsereignis eingetreten ist.

2. System nach Anspruch 1, wobei der eine oder mehreren Transaktionsdatensätze (602) weiter mit mindestens dem Teil der Aufzeichnung unter Verwendung
eines Überwachungsvorrichtungs-Identifikators einer Überwachungsvorrichtung (135A, 135B) der einen oder mehreren Überwachungsvorrichtungen (135A, 135B), die zumindest den Teil der Aufzeichnung aufgenommen hat, verknüpft sind.

3. System nach einem der Ansprüche 1 oder 2, wobei die Operationen weiter umfassen: Speichern an dem mindestens einen Ausgabeschrank (130) der verknüpften einen oder mehreren Transaktionsdatensätze (602) und mindestens des Teils der Aufzeichnung.

4. System nach einem der Ansprüche 1 bis 3, wobei die Vielzahl der Aufzeichnungen in einer Datenbank (120) gefiltert werden, die von dem mindestens einen Ausgabeschrank (130) entfernt ist.

5. System nach einem der Ansprüche 1 bis 4, wobei die Operationen weiter umfassen: Zugreifen in einer Datenbank (120), die von dem mindestens einen Ausgabeschrank (130) entfernt ist, auf den einen oder mehreren Transaktionsdatensätze (602) und die Aufzeichnung, die von dem einen oder mehreren Überwachungsvorrichtungen (135A, 135B) erfasst wurde.

6. System nach einem der Ansprüche 1 bis 5, wobei die Operationen weiter umfassen: Bestimmen basierend auf gefilterten Transaktionsdatensätzen und der Aufzeichnung, dass das mögliche Abzweigungsereignis eingetreten ist.

7. System nach einem der Ansprüche 1 bis 6, wobei das auslösende Ereignis eines oder mehrere umfasst von: eine Person, die ein Patientenzimmer betritt, eine Person, die sich für eine bestimmte Zeitspanne innerhalb einer vorbestimmten Nähe des mindestens einen Ausgabeschranks (130) befindet, und eine Person, die mit dem mindestens einen Ausgabeschrank (130) interagiert.

8. System nach einem der Ansprüche 1 bis 7, wobei die Aufzeichnung mindestens eine Video-, Bild- und Audioaufzeichnung umfasst.

9. System nach einem der Ansprüche 1 bis 8, wobei die Überwachungsvorrichtung (135A, 135B) an dem mindestens einen Ausgabeschrank (130) positioniert ist; und wobei eine zweite Überwachungsvorrichtung (135A, 135B) der einen oder mehreren Überwachungsvorrichtungen (135A, 135B) an einer Hilfsvorrichtung (170) positioniert ist, die mit dem mindestens einen Ausgabeschrank (130) gekoppelt ist.

10. System nach Anspruch 9, wobei das erfasste mögliche Abzweigungsereignis von einem Hilfsüberwachungsvorrichtungs-Hub (137A, 137B) an der Hilfsvorrichtung (170) an einen Hauptüberwachungsvorrichtungs-Hub (137) an dem mindestens einen Ausgabeschrank (130) übertragen wird.

11. System nach einem der Ansprüche 1 bis 10, wobei die Operationen weiter umfassen:
Erkennen, basierend auf der Aufzeichnung, einer Menge des Medikaments, das aus dem mindestens einen Ausgabeschrank (130) entnommen wurde; wobei die Bestimmung, dass das mögliche Abzweigungsereignis eingetreten ist, ein Bestimmen umfasst, dass die erkannte Menge des Medikaments sich von einer erwarteten Menge des Medikaments unterscheidet.

12. System nach Anspruch 1, wobei die Operationen weiter umfassen: Übertragen der verknüpften Transaktionsdatensätze (602) und mindestens des Teils der Aufzeichnung an eine entfernte Datenbank (120), um die Filterung in der Datenbank (120) zu ermöglichen.

13. System nach Anspruch 1, wobei das Erkennen des auslösenden Ereignisses die Erzeugung des einen oder mehrerer Transaktionsdatensätze (602) bewirkt.

14. System nach Anspruch 1, wobei das Verknüpfen des einen oder mehreren Transaktionsdatensätzen (602) mit mindestens dem Teil der Aufzeichnung, der das erfasste mögliche Abzweigungsereignis umfasst, ermöglicht, dass jeder der Teile der Aufzeichnung leichter durch die verknüpften Transaktionsdatensätze (602) gefiltert werden kann.

15. System nach Anspruch 11, wobei die Operationen weiter umfassen: Anfordern, dass die Menge des aus dem mindestens einen Ausgabeschrank (130) entnommenen Medikaments zumindest der Überwachungsvorrichtung (135A, 135B) gezeigt werden soll.

## Revendications

1. Système de distribution automatisée de médicaments, comprenant
au moins une armoire de distribution (130) contenant un médicament ;
un dispositif de surveillance (135A, 135B) dont la zone de détection comprend l'au moins une armoire de distribution (130) ;
au moins un processeur de données (810) ; et
au moins une mémoire (820) stockant des instructions qui, lorsqu'elles sont exécutées par l'au moins un processeur de données (810), aboutissent à des opérations comprenant :
détecter un événement déclencheur dans l'au moins une armoire de distribution (130) ;
activer, en réponse à la détection de l'événement déclencheur, un ou plusieurs dispositifs de surveillance (135A, 135B) pour capturer un enregistrement comprenant un événement de détournement possible dans l'au moins une armoire de distribution (130), dans lequel l'un ou plusieurs dispositifs de surveillance (135A, 135B) comprend le dispositif de surveillance (135A, 135B) ;
générer, sur la base de l'événement déclencheur, un ou plusieurs enregistrements de transaction (602) associés à l'enregistrement, dans lesquels l'un ou plusieurs enregistrements de transaction (602) comprennent un ou plusieurs de : un identifiant de clinicien, un identifiant de patient, un type de transaction, un identifiant de transaction, un identifiant de témoin et un type de médicament ;
relier l'un ou plusieurs enregistrements de transaction (602) à au moins une partie de l'enregistrement qui comprend l'événement de détournement possible capturé, dans lequel l'un ou plusieurs enregistrements de transaction (602) sont liés à au moins la partie de l'enregistrement en utilisant au moins un horodatage indiquant une heure de début et une heure de fin de l'événement déclencheur ;
filtrer une pluralité d'enregistrements incluant l'enregistrement comprenant l'événement de détournement possible pour identifier un ou plusieurs enregistrements comprenant l'événement de détournement possible, le filtrage étant basé sur l'un ou plusieurs enregistrements de transaction (602) liés à l'au moins une partie de l'enregistrement qui comprend l'événement de détournement possible capturé ;
déterminer que l'événement de détournement possible s'est produit sur la base de l'un ou plusieurs enregistrement identifié comprenant l'événement de détournement possible et l'un ou plusieurs enregistrements de transaction (602) liés à l'au moins une partie de l'enregistrement qui comprend l'événement de détournement possible capturé ; et
en réponse à la détermination que l'événement de détournement possible s'est produit, générer une alerte indiquant que l'événement de détournement possible s'est produit.

2. Système de la revendication 1, dans lequel l'un ou plusieurs enregistrements de transaction (602) sont en outre liés à au moins la partie de l'enregistrement en utilisant
un identifiant de dispositif de surveillance d'un dispositif de surveillance (135A, 135B) de l'un ou plusieurs dispositifs de surveillance (135A, 135B) qui ont capturé au moins la partie de l'enregistrement.

3. Système de l'une des revendications 1 ou 2, dans lequel les opérations comprennent en outre : stocker, dans l'au moins une armoire de distribution (130), l'un ou plusieurs enregistrements de transaction liés (602) et au moins la partie de l'enregistrement.

4. Système de l'une des revendications 1 à 3, dans lequel la pluralité d'enregistrements sont filtrés dans une base de données (120) éloignée de l'au moins une armoire de distribution (130).

5. Système de l'une des revendications 1 à 4, dans lequel les opérations comprennent en outre : accéder, dans une base de données (120) éloignée de l'au moins une armoire de distribution (130), à l'un ou plusieurs enregistrements de transaction (602) et à l'enregistrement capturé par l'un ou plusieurs dispositifs de surveillance (135A, 135B).

6. Système de l'une des revendications 1 à 5, dans lequel les opérations comprennent en outre : déterminer, sur la base des enregistrements de transaction filtrés et de l'enregistrement, que l'événement de détournement possible s'est produit.

7. Système de l'une des revendications 1 à 6, dans lequel l'événement déclencheur comprend un ou plusieurs de: une personne entrant dans une chambre de patient, une personne se trouvant dans une proximité prédéterminée de l'au moins une armoire de distribution (130) pendant une durée seuil, et une personne interagissant avec l'au moins une armoire de distribution (130).

8. Système de l'une des revendications 1 à 7, dans lequel l'enregistrement comprend au moins un enregistrement vidéo, image et audio.

9. Système de l'une des revendications 1 à 8, dans lequel le dispositif de surveillance (135A, 135B) est positionné sur l'au moins une armoire de distribution (130) ; et dans lequel un second dispositif de surveillance (135A, 135B) de l'un ou plusieurs dispositifs de surveillance (135A, 135B) est positionné sur un dispositif auxiliaire (170) couplé à l'au moins une armoire de distribution (130).

10. Système de la revendication 9, dans lequel l'événement de détournement possible capturé est transmis d'un concentrateur de dispositif de surveillance auxiliaire (137A, 137B) au dispositif auxiliaire (170) à un concentrateur de dispositif de surveillance principal (137) à l'au moins une armoire de distribution (130).

11. Système de l'une des revendications 1 à 10, dans lequel les opérations comprennent en outre :
détecter, sur la base de l'enregistrement, une quantité du médicament extraite de l'au moins une armoire de distribution (130) ; dans lequel, la détermination que l'événement de détournement possible s'est produit comprend déterminer que la quantité détectée du médicament est différente d'une quantité attendue du médicament.

12. Système de la revendication 1, dans lequel les opérations comprennent en outre : transmettre les enregistrements de transaction liés (602) et au moins la partie de l'enregistrement à une base de données éloignée (120) pour permettre le filtrage dans la base de données (120).

13. Système de la revendication 1, dans lequel la détection de l'événement déclencheur entraîne la génération de l'un ou plusieurs enregistrements de transaction (602).

14. Système de la revendication 1, dans lequel le fait de lier un ou plusieurs enregistrements de transaction (602) à au moins la partie de l'enregistrement qui comprend l'événement de détournement possible capturé permet à chacune des parties de l'enregistrement d'être filtrée plus facilement par les enregistrements de transaction associés (602).

15. Système de la revendication 11, dans lequel les opérations comprennent en outre : demander que la quantité du médicament extraite de l'au moins une armoire de distribution (130) soit montrée à au moins le dispositif de surveillance (135A, 135B).
